# EUROPEAN PATENT APPLICATION

(11) **EP 3 785 798 A1**
(43) Date of publication of application: **03.03.2021**
(21) Application number: 19193634.3
(22) Date of filing: 26.08.2019
(51) Int. Cl.: B01L 3/00, G01N 1/31, H01F 1/44, A61B 10/00

(54) **A DYNAMIC FERROFLUID BARRIER FOR A BIOLOGICAL SAMPLE**

(71) Applicant: Koninklijke Philips N.V., 5656 AG Eindhoven (NL)
(72) Inventor: VULDERS, Roland Cornelis Martinus, 5656 AE Eindhoven (NL); BOAMFA, Marius Iosif, 5656 AE Eindhoven (NL); VAN DER ZAAG, Pieter Jan, 5656 AE Eindhoven (NL); ASSELMAN, Michel Jozef Agnes, 5656 AE Eindhoven (NL)
(74) Representative: de Haan, Poul Erik

(57) **Abstract**

In order to improve the effectiveness of processing a 3D tissue sample (16), a method is provided that uses a dynamic seal (18), e.g. a seal formed of ferrofluids or other magnetorheological fluids, as a physical barrier for preventing leakage of a tissue processing fluid (24), e.g. a staining medium, around the 3D tissue sample (16) received in a tube (10), thereby forcing the tissue processing fluid (24) to flow through the 3D tissue sample (16). The dynamic seal (18) is a liquid made of micro- and/or nanoscale particles suspended in a carrier fluid that is characterized by its increase in viscosity when subjected to a sufficiently strong external field such as a magnetic field generated by a permanent magnet (20).

## Description

### FIELD OF THE INVENTION

The present invention relates to a method and a system for processing a 3D tissue sample. The present invention further relates to a computer program element.

### BACKGROUND OF THE INVENTION

For the proper analysis of tumors and defining the appropriate treatment, detailed information on a tumor may be needed. First, the presence and position of a potential tumor may be identified through medical imaging.

Subsequently, a biopsy may be taken to assess whether or not the lesion is benign or malignant through pathology. For positioning the biopsy device accurately in the suspicious tissue, the correct location is commonly determined using image guidance such as Ultrasound, Magnetic Resonance Imaging (MRI), or X-ray. While imaging may provide coarse guidance of the needle towards the region of interest, it is often challenging to identify precisely the boundaries of small lesions or tumors with the biopsy needle using standard imaging modalities. Consequently, biopsies may occasionally be taken at the wrong location, which may increase the risk of incorrect diagnoses. Following the extraction of biopsy tissue out of a body, the tissue is subjected to for example a staining fluid in an open receptacle.

Finally, molecular diagnostic (MDx) analysis of the tissue may be done to determine which mutations and molecular pathway(s) drive the tumor in order to arrive at a proper treatment. In order to provide the correct molecular analysis, also tumor heterogeneity may be assessed to determine whether a single cancerous clone is responsible for the tumorous growth or whether multiple clones are present, so that possibly multiple biological pathways drive tumor growth and a combination of drugs should be provided.

In the case of neo-adjuvant treatment, especially in case of a large tumor and in case of treatment with targeted drugs that target signal transduction pathways, the first diagnostic biopsy can be handled in a standard manner, however additional biopsies are needed to assess the heterogeneity of the tumor with respect to the underlying biology.

Furthermore, it may be a problem that typically staining of a three-dimensional (3D) tissue sample, i.e. of a not-sliced or complete thick tissue sample, takes comparatively long as staining occurs through diffusion. WO 2018/162640 A1 proposes to use a tube for retaining a 3D tissue sample such that the staining is performed actively under pressure, thereby resulting in much faster penetration of e.g. staining agents into the tissue and thus provide faster staining, as well as faster washing procedures. Moreover, this approach may also be advantageously used for 3D tissue samples, such as biopsy samples, that have been obtained in a conventional way and will be inserted in a tube for faster sample preparation e.g. staining. However, the 3D tissue sample usually has an irregular shape. Thus, spaces may exist between the 3D tissue sample and the channel wall of the tube. The flow of a fluid of choice, e.g., staining or washing medium, may tend to go through these spaces instead of the sample of interest, since the extra spaces become the path with lowest resistance. Thus, the staining process may be less efficient.

### SUMMARY OF THE INVENTION

There may be a need to improve the effectiveness of processing a 3D tissue sample.

The object of the present invention is solved by the subject matter of the independent claims, wherein further embodiments are incorporated in the dependent claims. It should be noted that the following described aspects of the invention apply also for the method, the system, and the computer program element.

A first aspect of the present invention provides a method of processing a 3D tissue sample. The method comprises the step of receiving a tube with an inner space and at least two open ends comprising a first open end and a second open end. The tube is adapted for retaining a 3D tissue sample and a dynamic seal in the inner space. The dynamic seal is a liquid made of micro- and/or nanoscale particles suspended in a carrier fluid that is characterized by its increase in viscosity when subjected to an external field. The method further comprises the step of applying a sufficiently strong external field to the dynamic seal to cause the dynamic seal to become a viscoelastic solid such that an interspace between the 3D tissue sample and an inner surface of the tube is sufficiently sealed along a length of the 3D tissue sample. The method further comprises the step of forcing a tissue processing fluid into the first open end of the tube, through the 3D tissue sample in the inner space of the tube, and out of the second open end of the tube.

In other words, a main aspect may be seen in that a tissue processing method is provided with a dynamic seal based on "smart fluids" that change their properties reversibly and fast (e.g., milliseconds) under presence of an external field. This field may be an electric, magnetic or electromagnetic field. These fluids can show changes in apparent viscosity of several orders of magnitude when an electric field, a magnetic field, or an electromagnetic field is applied, to the point of becoming a viscoelastic solid. For example, the dynamic seal may utilize fluids whose viscosity increases when a magnetic field is applied. Small magnetic dipoles are suspended in a non-magnetic fluid, and the applied magnetic field causes these small magnets to line up and form strings that increase the viscosity. Thus, when a 3D tissue sample, such as a biopsy sample or a resection sample, is suspended in a solution containing the dynamic seal and is loaded into a tube, the application of a sufficiently strong magnetic field will change the physical state of the dynamic seal from liquid to a solid, thereby generating an impermeable seal around the 3D tissue sample. Thus, the extra spaces between the 3D tissue sample and the inner surface of the tube will be sufficiently sealed along the length of the 3D tissue sample. The front- and the back-ends of the 3D tissue sample may not be part of the sealed region as otherwise the tissue processing fluids cannot flow through the 3D tissue sample. Consequently, by applying a flow containing a tissue processing fluid, e.g., a staining agent, this tissue processing fluid will be forced to permeate the 3D tissue sample since this becomes the path with lowest resistance.

As used herein, the term "sufficiently" refers to the complete or nearly complete extent or degree of a state as indicated. For example, a reference that the extra spaces are sufficiently sealed along the length of the 3D tissue sample would mean that these extra spaces are sufficiently sealed to carry out a particular function, i.e. preventing the tissue processing fluids from circumventing the 3D tissue sample. If for instance it would only seal the middle of the 3D tissue sample, both ends would not adequately be stained as tissue processing fluids could still go by the sides. The exact allowable degree of deviation from absolute completeness may depend on the desired tissue processing result. For example, in order to achieve a better tissue processing result, 90%, 95%, or 100% extra spaces along the length of the 3D tissue sample may be sealed.

As used herein, the term "length of the 3D tissue sample" refers a longitudinal dimension, meaning in the direction of the long axis of the 3D tissue sample. As the 3D tissue sample is retained within the tube and extends along a length of the tube, the long axis of the 3D tissue sample is substantially parallel to the long axis of the tube.

As used herein, the term "sufficiently sealed along a length of the 3D tissue sample" refers to a seal extending along an entire or nearly entire length of the 3D tissue sample for sealing the interspace between the inner surface of the tube and the 3D tissue sample. This may be 80%, 85%, 90%, 95%, or 100% of the entire length of the 3D tissue sample. The front- and the back-ends, i.e. the ends facing the first and second open ends, of the 3D tissue sample may not be part of the sealed region as otherwise the tissue processing fluids cannot flow through the 3D tissue sample.

As used herein, the term "tube" encompasses any container with at least two openings and an inner space. In particular, the used term does not prescribe any shape of the cross section or any dimensions. For example, the openings may be located at the ends opposite to each other. A direction from one open end to another open end may be denoted as longitudinal direction. In some examples, at least one opening may be located in a side surface of the tube. The inner space of the tube may be adapted to receive and retain a 3D tissue sample for processing the same. It can also be adapted to be used in/with a biopsy needle, such as the biopsy needle disclosed in WO 2016/075061 A1. The tube is then used to extract a tissue sample out of a patient's body so that the tissue is already in the tube when the tube is placed in the tube retainer for processing the tissue inside the tube. However, the tissue may also be inserted into the tube outside the patient's body. Preferably, the tissue is placed in the tube such that no or at least limited leakage of tissue processing fluid may occur in the tube.

According to an embodiment of the present invention, the applied external field is a magnetic field.

A permanent magnet and/or an electromagnet may be used for generating the magnetic field.

According to an embodiment of the present invention, the micro- and/or nanoscale particles comprise a magnetic material comprising at least one of: a ferromagnetic material, a ferrimagnetic material, a synthetic magnetic material, and a paramagnetic material.

In other words, a magnetic material is made into particles such that the relevant temperature, preferably in a range between room temperature and 40 °C, is so small that the net magnetic moment is not thermodynamically stable and is fluctuating in space, because its magnetostatic energy is less than the thermal energy. Application of a magnetic field will stabilize the magnetic moment direction of all particles and have the particles cluster together, thereby increasing the viscosity, to the point of becoming a viscoelastic solid.

For example, the dynamic seal may be suspensions of finely divided, e.g., less than 10 µm in diameter, low coercivity, magnetisable particles of a material such as iron, nickel, cobalt, gadolinium, and their magnetic alloys dispersed in a base liquid or liquid vehicle such as a mineral oil, synthetic hydrocarbon, water, silicone oil, esterified fatty acid or other suitable organic liquid. The dynamic seal has an acceptably low viscosity in the absence of a magnetic field but display large increases in their dynamic yield stress when they are subjected to a magnetic field.

According to an embodiment of the present invention, the magnetic material is in a superparamagnetic state.

Superparamagnetism is a property occurring principally in small, single-domain magnetic particles without magnetic memory. It is more closely related to ferromagnetism than to paramagnetism. For example, when a ferromagnetic, multi-domain sample of Fe₃O₄ is reduced in size to less than about 40 nm, a single-domain magnetic particle eventually will be formed. When placed in an external magnetic field, this particle develops a strong internal magnetization from exchange coupling of spins within the domain and thus becomes superparamagnetic. Since only a single domain is involved, the magnetic moment of a superparamagnetic substance is not nearly as great as that of a ferromagnetic substance of a larger sample. Additionally, since each (small) particle is in a single domain, leading to a fluctuating moment of each particle when the thermal energy exceeds the magneto-static energy. Therefore, there can be no interactions to ordering of domains within a sample, which are made of these single domain superparamagnetic particles. Unlike ferromagnetic materials, therefore, superparamagnetic substances do not retain any net overall magnetization, once the external field has been removed. In other words, they have no magnetic memory. For a detailed discussion concerning the superparamagnetic material, reference is made to the following publication: George T. Rado and Harry Suhl, Magnetism. New York and London: Academic Press, 1963, Vol. III, pages 271 to 294.

An advantage of using superparamagnetic particles may be seen in that the removal of the magnetic field can release the fixation of the 3D tissue sample without any delay or with less delay as the superparamagnetic particles have no magnetic memory. This may be beneficial for enabling further processing of the sample for conventional two-dimensional pathology.

According to an embodiment of the present invention, the dynamic seal comprises a ferrofluid.

A ferrofluid relates to a liquid, which becomes strongly magnetized in the presence of a magnetic field. Typically, ferrofluids are colloidal liquids made of nanoscale ferromagnetic, or ferromagnetic particles suspended in a carrier fluid, such as an organic solvent or water. Each tiny particle may be thoroughly coated with a surfactant to inhibit aggregation. When the external field is removed, ferrofluids do not retain magnetization and are therefore classified as superparamagnetic substances. Ferrofluid seals are known to work with liquid seal applications. For a detailed discussion concerning the use of a ferrofluid seal against a liquid, reference is made to the following publication: R. A. Williams and H. Malsky, "Some experiences using a ferrofluid seal against a liquid", IEEE Transactions on Magnetics, Vol. MAG-16, No. 2, pages 379 to 381, March 1980.

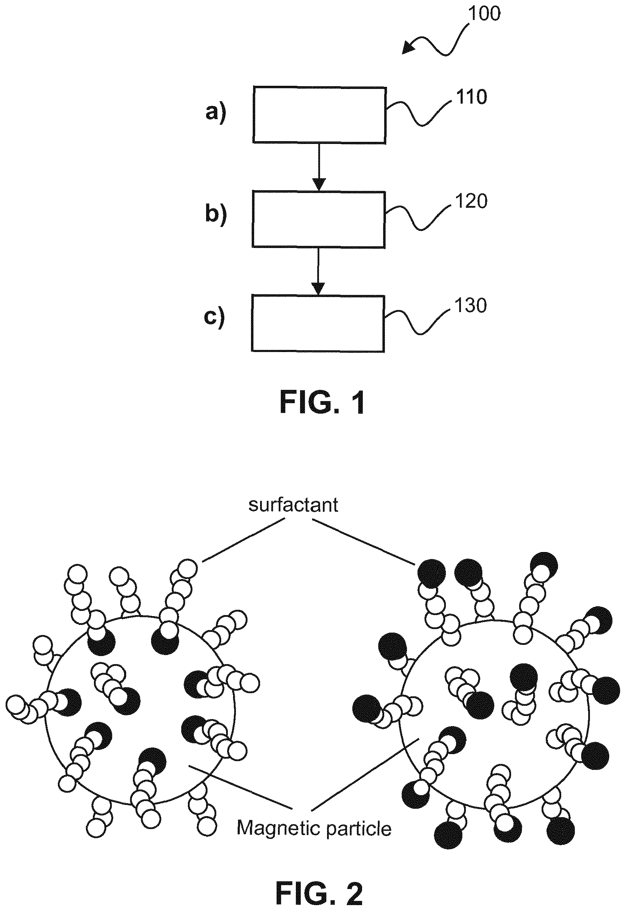

An advantage of using ferrofluid seals as the dynamic seal for processing a 3D tissue sample may be seen in that the application of a magnetic field can fixate the 3D tissue sample and provide an impermeable seal around the specimen for forcing the tissue processing liquid to flow through the 3D tissue sample, whereas the removal of the magnetic field can release the 3D tissue sample without any delay (or with less delay) for enabling further conventional two-dimensional (2D) processing. An example of the ferrofluids is illustrated in FIG. 2.

According to an embodiment of the present invention, the carrier fluid comprises at least one of water, oil, an organic solvent and an ionic liquid.

According to an embodiment of the present invention, the tissue processing fluid comprises at least one of a clearing agent and a staining agent.

A further aspect of the present invention provides a system for processing a 3D tissue sample. The system comprises a tube, which has at least two open ends comprising a first open end and a second open end and an inner space for accommodating the 3D tissue sample and a dynamic seal. The dynamic seal is a liquid made of micro- and/or nanoscale particles suspended in a carrier fluid that is characterized by its increase in viscosity when subjected to an external field. The system also comprises a tube retainer for holding the tube. The system further comprises a source for applying a sufficiently strong external field to the dynamic seal to cause the dynamic seal to become a viscoelastic solid such that an interspace between the 3D tissue sample and an inner surface of the tube is sufficiently sealed along a length of the 3D tissue sample. The system further comprises a pumping device for supplying the tissue processing fluid to the tube to force the tissue processing fluid into the first open end of the tube, through the 3D tissue sample in the inner space of the tube, and out of the second open end of the tube.

According to an embodiment of the present invention, the source comprises a magnet including at least one of a permanent magnet and an electromagnet.

According to an embodiment of the present invention, the micro- and/or nanoscale particles comprise a magnetic material comprising at least one of: a ferromagnetic material, a ferrimagnetic material, a synthetic magnetic material, and a paramagnetic material.

According to an embodiment of the present invention, the magnetic material is in a superparamagnetic state.

According to an embodiment of the present invention, the dynamic seal comprises a ferrofluid.

According to an embodiment of the present invention, the tissue processing fluid includes at least one of a clearing agent and a staining agent.

According to an embodiment of the present invention, the system further comprises a reservoir for providing a tissue processing fluid and/or a dynamic seal, and a supply line for connecting the reservoir and the first end of the tube, such that the tissue processing fluid and/or the dynamic seal can flow from the reservoir, through the supply line into the first open end of the tube.

A further aspect of the present invention provides a computer program element that comprises sets of instructions, wherein, when the sets of instructions are executed on a processing unit of the system of the above and below described embodiments and examples, the sets of instructions cause the system to perform the method of the above and below described embodiments and examples.

A further aspect of the present invention provides a computer readable medium having stored the program element.

Summarizing, in order to improve the effectiveness of processing a 3D tissue sample, a method and a system are provided that use a dynamic seal as a physical barrier preventing leakage of sheath fluid around a biological specimen, thereby forcing the sheath fluid to enter the biological sample. Examples of the dynamic seal may include electrorheological fluids, magnetorheological fluids, or ferrofluids.

These and other aspects of the present invention will become apparent from and be elucidated with reference to the embodiments described hereinafter.

### BRIEF DESCRIPTION OF THE DRAWINGS

These and other aspects of the invention will be apparent from and elucidated further with reference to the embodiments described by way of examples in the following description and with reference to the accompanying drawings, in which
FIG. 1 is a flowchart representative of a method of processing a 3D tissue sample.
FIG. 2 shows a schematic representation of a ferrofluid with a nanoparticle coated with a surfactant.
FIGs. 3A and 3B show an exemplary implementation of a magnetic field.
FIGs. 4A and 4B show another exemplary implementation of a magnetic field.
FIG. 5 depicts a block diagram of an exemplary implementation of a system for processing a 3D tissue sample

It should be noted that the figures are purely diagrammatic and not drawn to scale. In the figures, elements, which correspond to elements already described, may have the same reference numerals. Examples, embodiments or optional features, whether indicated as non-limiting or not, are not to be understood as limiting the invention as claimed.

### DETAILED DESCRIPTION OF EMBODIMENTS

FIG. 1 is a flowchart representative of a method 100 of processing a 3D tissue sample.

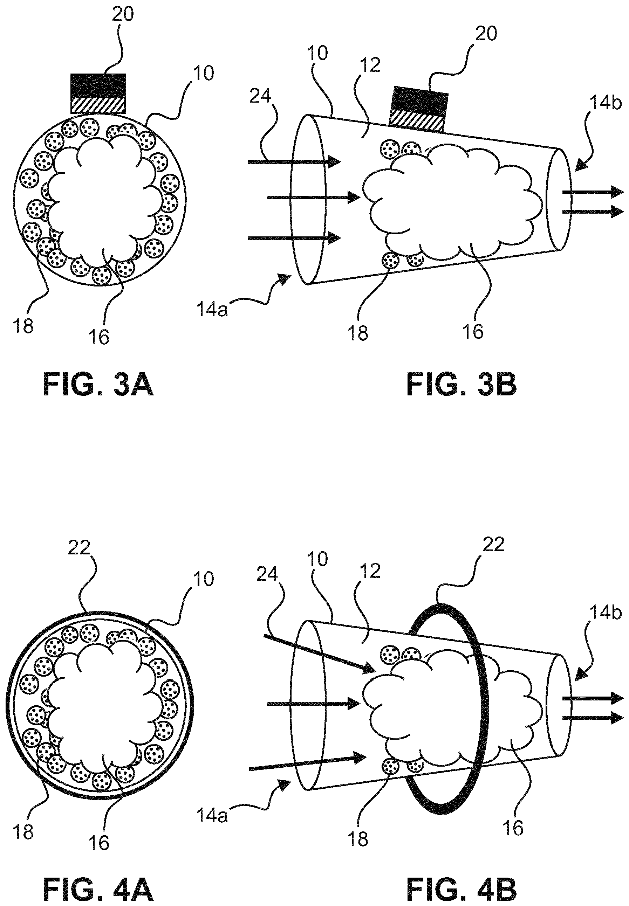

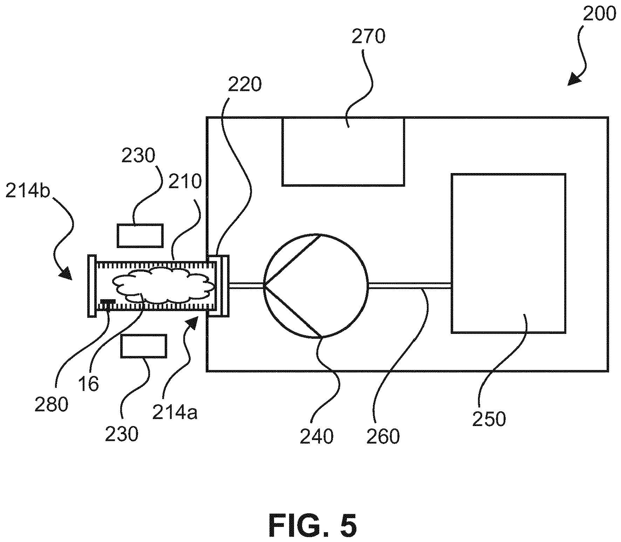

In step 110, i.e. step a), a tube 10 is received, e.g., engaged with a staining station as illustrated in FIG. 5. An exemplary implementation of the tube is described in conjunction with FIGs. 3A and 3B and FIGs. 4A and 4B. The tube 10 of the illustrated examples in FIGs. 3A and 3B and FIGs. 4A and 4B has an inner space 12 and at least two open ends 14 comprising a first open end 14a and a second open end 14b. The illustrated example tube 10 has two open ends opposite to each other. In some other examples (not shown), at least one opening may be located in a side surface of the tube. The tube 10 is adapted for retaining a 3D tissue sample 16 and a dynamic seal 18 in the inner space 12. The dynamic seal 18 is a liquid made of micro- and/or nanoscale particles suspended in a carrier fluid that is characterized by its increase in viscosity when subjected to a sufficiently strong external field, to the point of becoming a viscoelastic solid.

Examples of the micro- and/or nanoscale particles may include electrorheological fluids, magnetorheological fluids, or ferrofluids. In the presence of an appropriate electric field, magnetic field or electromagnetic field, solid particles in the dynamic seal move into alignment. When this alignment occurs, the ability of the fluid to flow, or be sheared, is substantially decreased. In the presence of an energy field, the dynamic seal responds by forming fibrous structures parallel to the applied field. The formation of these fibrous structures triggers a significant increase in the viscosity of the fluid, by factors as high as 10⁵.

Optionally, the micro- and/or nanoscale particles may comprise a magnetic material, such as a ferromagnetic material, a ferrimagnetic material, a synthetic magnetic material, and/or a paramagnetic material. Superparamagnetic particles may be preferred as they have no magnetic memory. Thus, the removal of the magnetic field can trigger the release of the fixation of the 3D tissue sample, enabling further conventional 2D processing. Possible delay due to the magnetic memory may be avoided.

As previously mentioned, it is preferred to have particles with a small size such that the net magnetic moment is not thermodynamically stable and is fluctuating in space, because its magnetostatic energy is less than the thermal energy. The micro- and/or nanoscale particles may have an average size of 2 nm to 10 µm, preferably from 3 nm to 200 nm, more preferably from 5 nm to 100 nm, such as 10 nm.

The micro- and/or nanoscale particles may be dispersed in any suitable carrier fluid (or solvent), such as water, oil, an organic solvent and an ionic liquid.

Preferably, the dynamic seal comprises a ferrofluid. Ferrofluids are nanoscale particles (e.g., 100 nm or less in diameter) suspended in a carrier fluid, which contains a surfactant thereby preventing agglomeration of the particles. For a detailed discussion concerning how the single domain size changes as a function of the magnetic character of the environment, reference is made to the following publication: P. J. van der Zaag, et. al., "The initial permeability of polycrystalline MnZn ferrites: The influence of domain and microstructure", J. Appl. Phys. Vol. 74, No. 6, pages 4085 to 4095, September 1993. In the absence of a magnetic field, the particles are sufficiently small to remain in suspension due to Brownian motion. This is in contrast to magnetorheological fluids, which, in the absence of a magnetic field, settle or phase separate. The ideal ferrofluid does not phase separate while exposed to a (strong) magnetic field. A surfactant, consisting of a polar head and a non-polar tail or vice versa, one of which has to absorb to the nanoparticle or covalently be bound to the nanoparticle, is used to make these particles soluble (water or oil). FIG. 2 shows a schematic representation of a ferrofluid with a nanoparticle coated with a surfactant. When a magnetic field is applied, the properties of a fluid remain and thus the particles can be manipulated by magnets such as movement of the liquid. Particles in ferrofluids are dispersed in a liquid, using a surfactant, and thus ferrofluids are colloidal suspensions. For example, the composition of a ferrofluid may consist of 5% magnetic solids, 10% surfactant and 85% carrier. The magnetic core may be an iron-oxide such as magnetite (Fe₃O₄) -like. Ferrofluids may also exist in which the core is a Fe particle with a coating to prevent oxidation. Changes in the state of matter can be performed by applying a magnetic field. When "activated" the fluid solidifies and returns to its fluid state when the magnetic field is removed.

In step 120, i.e. step b), a sufficiently strong external field, e.g. an electric field or a magnetic field, is applied to the dynamic seal to cause the dynamic seal to solidify such that an interspace between the 3D tissue sample and the tube is sufficiently sealed along a length of the 3D biopsy sample. The front- and the back-ends of the 3D tissue sample may not be part of the sealed region as otherwise the tissue processing fluids cannot enter the 3D tissue sample.

An exemplary implementation of the magnetic field is described in conjunction with FIG. 3A, which illustrates a cross sectional view of the tube 10. In this illustrated example, the magnetic field is generated by a permanent magnet 20. If an irregular formed biological specimen, which is suspended in the dynamic seal 18, is loaded into the tube 10 and reaches the permanent magnet 20, the dynamic seal becomes activated and starts to solidify (provided that the magnetic field is sufficiently strong). The solidified dynamic seal can fixate the sample and provides an impermeable seal around the specimen.

Another exemplary implementation of the magnetic field is described in conjunction with FIG. 4A, which also illustrates a cross sectional view of the tube 10. In this illustrated example, the magnetic field is generated by an electromagnet 22 surrounding the tube 10. The electromagnet 22 may allow a user to control over the state of the ferrofluid. Moreover, as the electromagnet 22 surrounds the tube 10, the magnetic field may be optimized and further manipulated in comparison to a static magnet as illustrated in FIGs. 3A and 3B. For example, quadrupole magnets may be used to tune the desired dynamic seal. The solidified dynamic seal 18 can fixate the sample and providing an impermeable seal around the specimen 16.

In step 130, i.e. step c), a tissue processing fluid is forced into the first open end of the tube, through the 3D tissue sample in the inner space of the tube, and out of the second open end of the tube.

An exemplary implementation of the tissue processing fluid is described in conjunction with FIG. 3B, which illustrates a side view of the tube 10. In this illustrated example, the tissue processing fluid 24 (indicated with an arrow), such as staining or washing medium, is forced into the first open end 14a of the tube 10. In staining procedures, it is essential that the flow of a fluid of choice goes through the sample of interest. An advantage of using the dynamic seal may be seen in that an impermeable seal is formed in the presence of the magnetic field generated by the permanent magnet 20, such that the extra spaces between the sample 16 and the channel wall of the tube 10 are sealed. Thus, the tissue processing fluid is forced to permeate the sample 16 since this becomes the path with lowest resistance. Thus, the effectiveness of processing the 3D tissue sample is improved.

Another exemplary implementation of the tissue processing fluid is described in conjunction with FIG. 4B, which illustrates a side view of the tube 10. In this illustrated example, the magnetic field is generated by the electromagnet 22, which solidifies the dynamic seal 18 to form an impermeable seal around the sample 16. With the impermeable seal, the tissue processing fluid can only permeate the sample 16, as this becomes the path with lowest resistance. Thus, the effectiveness of processing the 3D tissue sample is improved.

FIG. 5 is a block diagram of an exemplary implementation of a system 200 for processing a 3D tissue sample. The exemplary system comprises a tube 210, a tube retainer 220, a source 230 for applying an external field, and a pumping device 240. The tube 210 has at least at least two open ends 214 comprising a first open end 214a and a second open end 214b and an inner space for accommodating the 3D tissue sample 16 and a dynamic seal 18. The dynamic seal is a liquid made of micro- and/or nanoscale particles suspended in a carrier fluid that that is characterized by its increase in viscosity when subjected to a sufficiently strong external field. The micro- and/or nanoscale particles may comprise a magnetic material comprising at least one of: a ferromagnetic material, a ferrimagnetic material, a synthetic magnetic material, and a paramagnetic material. Preferably, the magnetic material is in a superparamagnetic state, as the superparamagnetic particles have no magnetic memory. More preferably, the dynamic seal comprises a ferrofluid.

The first open end 214a of the tube 210 may engage with the tube retainer 220. Although it is shown in FIG. 5 that the tube 210 is retained at its end portion in the tube retainer 220, it will be understood, that the tube retainer 220 may also engage the tube 210 at any other portion of the tube. In any case, the second open end 214b allows air or fluid to exit the tube as soon as the liquid or fluid which is supplied under pressure or under application of vacuum as driving force at the tube retainer 220 to the first open end 214a of the tube 210.

The source 230 may be configured to apply an electric field or a magnetic field to the dynamic seal inside the tube 210. Two exemplary implementations of a source of the magnetic field are illustrated in FIGs. 3A and 3B and FIGs. 4A and 4B. If the applied external field, e.g., magnetic field or electric field, is sufficiently strong, the dynamic seal can show changes in apparent viscosity of several orders of magnitude, to the point of becoming a viscoelastic solid. The solidified dynamic seal thus provides an impermeable seal filling the interspace between the 3D tissue sample 16 and the tube 210.

The pumping device 240 is configured for supplying the tissue processing fluid under pressure or under application of vacuum as driving force to the tube 210 to force the tissue processing fluid into the first open end 214a of the tube, through the 3D tissue sample 16 in the inner space of the tube, and out of the second open end 214b of the tube 210. For example, the liquid or fluid may be suitable for clearing the cellular structures of the tissue. Otherwise, the fluid or liquid may be for staining the tissue. The system 200 may optionally comprise a reservoir 250 for providing a tissue processing fluid and a supply line 260 for connecting the reservoir 250 and the first end 14a of the tube 210. When activated, the pumping device 240 will draw at its entering side the liquid or fluid from the reservoir 250 into the supply line 260, and will supply the liquid or fluid under pressure on its exit side to the biopsy tube 210. Alternatively, the pumping device may use vacuum as driving force to supply the liquid and fluid on its exit side to the biopsy tube. Optionally, a plurality of reservoirs (not shown) may be provided for a plurality of tissue processing fluids and at least one dynamic seal. A fluid manifold (not shown) may be provided to connect one of the plurality of reservoir to the supply line.

Before processing a 3D tissue sample, the pumping device 240 may be configured to draw at its entering side the dynamic seal from the reservoir 250 into the supply line 260, and will supply the dynamic seal to the tube 210 such that the 3D tissue sample 16 is surrounded by the dynamic seal such that an interspace between the 3D tissue sample and an inner surface of the tube is sufficiently sealed along a length of the 3D tissue sample. Before applying the tissue processing fluid, any leftover ferrofluid at both the front and the end side of the 3D tissue samples need to be removed. For this purpose, a valve may be provided for controlling the release of dynamic seal. An exemplary outlet valve 280 is illustrated in FIG. 5, which is arranged at the back-end of the biopsy tube 210 close to the second open end 214b such that the ferrofluid may be simply pumped away, e.g., when going to the next step of processing the 3D tissue sample. A further outlet valve (not shown) may be provided and arranged at the front-end of the biopsy tube 210 close to the first open end 214a for improving the efficiency of pumping away the dynamic seal from the front-end of the biopsy tube 210.

All steps described in connection with the method of processing a 3D tissue sample may be standardized and thus performed automatically, e.g., with the system 200 of the illustrated example in FIG. 5. When considering a computer-controlled handling device, even step a), i.e. the insertion of the biopsy tube 210 into the tube retainer 220 may be performed automatically. In addition, step b), i.e. the application of an external electric or magnetic field to the dynamic seal may also be performed automatically. For example, if a permanent magnet 20 of the illustrated example in FIGs. 3A and 3B is used, a retractable arm (not shown) may be provided to hold the permanent magnet 20. The retractable arm may be controlled by a processing unit 270 to move the permanent magnet 20 close to the tube 210 for applying the magnetic field, and to move the permanent magnet 20 away from the tube 210 for removing the magnetic field. On the other hand, if an electromagnet 22 of the illustrated example in FIGs. 4A and 4B is used, a switch may be provided under the control of the processing unit 270 to switch on the electromagnet 22 for applying a magnetic field, and to switch off the electromagnet 22 for stopping the application of the magnetic field. A computer program element including sets of instructions and being executable on the processing unit 270, may be provided on a suitable medium such as an optical storage medium or a solid-state medium supplied together with or as part of the processor, but may also be distributed in other forms such as via the internet or other wired or wireless telecommunication systems. When executed on the processing unit 270 the sets of instructions of the computer program element may cause the device for processing the 3D tissue sample to perform the above described method steps in an appropriate sequence.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfil the functions of several items re-cited in the claims. The mere fact that certain measures are re-cited in mutually different dependent claims does not indicate that a combination of these measures cannot be used to advantage. Any reference signs in the claims should not be construed as limiting the scope.

## Claims

1. A method (100) of processing a three-dimensional, 3D, tissue sample, comprising the steps of:
a) receiving (110) a tube (10, 210) with an inner space (12) and at least two open ends comprising a first open end (14a, 214a) and a second open end (14b, 214b), wherein the tube is adapted for retaining a 3D tissue sample (16) and a dynamic seal (18) in the inner space, wherein the dynamic seal is a liquid made of micro- and/or nanoscale particles suspended in a carrier fluid that is **characterized by** its increase in viscosity when subjected to an external field;
b) applying (120) a sufficiently strong external field to the dynamic seal to cause the dynamic seal to become a viscoelastic solid such that an interspace between the 3D tissue sample and an inner surface of the tube is sufficiently sealed along a length of the 3D tissue sample; and
c) forcing (130) a tissue processing fluid (24) into the first open end of the tube, through the 3D tissue sample in the inner space of the tube, and out of the second open end of the tube.

2. Method according to claim 1,
wherein the applied external field is a magnetic field.

3. Method according to claim 2,
wherein the micro- and/or nanoscale particles comprise a magnetic material comprising at least one of:
- a ferromagnetic material;
- a ferrimagnetic material;
- a synthetic magnetic material; and
- a paramagnetic material.

4. Method according to claim 3,
wherein the magnetic material is in a superparamagnetic state.

5. Method according to claim 4,
wherein the dynamic seal comprises a ferrofluid.

6. Method according to any one of the preceding claims,
wherein the carrier fluid comprises at least one of water, oil, an organic solvent and an ionic liquid.

7. Method according to any one of the preceding claims,
wherein the tissue processing fluid comprises at least one of a clearing agent and a staining agent.

8. A system (200) for processing a three-dimensional, 3D, tissue sample, comprising:
- a tube (10, 210), the tube having at least two open ends (14, 214) comprising a first open end (14a, 214a) and a second open end (14b, 214b) and an inner space (12) for accommodating the 3D tissue sample and a dynamic seal, wherein the dynamic seal is a liquid made of micro- and/or nanoscale particles suspended in a carrier fluid that is **characterized by** its increase in viscosity when subjected to an external field;
- a tube retainer (220) for holding the tube;
- a source (230) for applying a sufficiently strong external field to the dynamic seal to cause the dynamic seal to become a viscoelastic solid such that an interspace between the 3D tissue sample and an inner surface of the tube is sufficiently sealed along a length of the 3D tissue sample; and
- a pumping device (240) for supplying the tissue processing fluid to the tube to force the tissue processing fluid into the first open end of the tube, through the 3D tissue sample in the inner space of the tube, and out of the second open end of the tube.

9. System according to claim 8,
wherein the source comprises a magnet including at least one of a permanent magnet (20) and an electromagnet (22).

10. System according to claim 9,
wherein the micro- and/or nanoscale particles comprise a magnetic material comprising at least one of:
- a ferromagnetic material;
- a ferrimagnetic material;
- a synthetic magnetic material; and
- a paramagnetic material.

11. System according to claim 10,
wherein the magnetic material is in a superparamagnetic state.

12. System according to claim 11,
wherein the dynamic seal comprises a ferrofluid.

13. System according to any one of claims 8 to 12,
wherein the tissue processing fluid includes at least one of a clearing agent and a staining agent.

14. System according to any one of claims 8 to 13, further comprising:
- a reservoir (250) for providing a tissue processing fluid and/or a dynamic seal; and
- a supply line (260) for connecting the reservoir and the first end of the tube, such that the tissue processing fluid and/or the dynamic seal can flow from the reservoir, through the supply line into the first open end of the tube.

15. A computer program element comprising sets of instructions, wherein, when the sets of instructions are executed on a processing unit (270) of the system of claim 8, the sets of instructions cause the system to perform the method of claim 1.
